# EUROPEAN PATENT APPLICATION

(11) **EP 4 335 358 A1**
(43) Date of publication of application: **13.03.2024**
(21) Application number: 22194743.5
(22) Date of filing: 09.09.2022
(51) Int. Cl.: A61B 5/00, A61B 34/20

(54) **REGISTERING AN ANATOMICAL MODEL TO A REFERENCE ANATOMICAL MODEL**

(71) Applicant: Koninklijke Philips N.V., 5656 AG Eindhoven (NL)
(72) Inventor: FERNANDO, Shakith Devinda, Eindhoven (NL); VAN BREE, Karl Catharina, Eindhoven (NL)
(74) Representative: Philips Intellectual Property & Standards

(57) **Abstract**

A mechanism for registering an anatomical model, generated from location measurements, to a reference anatomical model. A path taken by an interventional device (for which the location measurements are obtained) is determined. The determined path is used to register the anatomical model to the reference anatomical model.

## Description

### FIELD OF THE INVENTION

The present invention relates to the field of medical imaging, and in particular, to anatomical models of anatomical cavities.

### BACKGROUND OF THE INVENTION

Medical imaging is an important aspect in diagnosing and/or treating a subject. There is an increasing interest in the use of device tracking imaging, which generates an anatomical model of an anatomical cavity using one or more elements carried by an interventional device.

In device tracking imaging, an interventional device is maneuvered within an anatomical cavity and location data, responsive to the location of the interventional device, is obtained. The location data can then be processed to construct an anatomical model of the internal anatomy or anatomical cavity of the subject, e.g., by constructing a mesh around recorded locations/positions defined by the location data. This is achievable because it can be generally assumed that the interventional device will only be located within cavities of the subject, thereby allowing the bounds of the cavity to be constructed/inferred.

The location data will comprise a plurality of location measurements, each location measurement being dependent upon a position of the interventional device within the anatomical cavity. Suitable examples of location measurements include: the electrical response of an electrode carried by the interventional device to crossing electric fields induced within the anatomical cavity; the magnetic response (e.g., of a Hall-effect sensor carried by the interventional device) to one or more magnetic fields induced within the anatomical cavity and so on. Yet other approaches include ultrasound-based tracking and the Fiber Optic RealShape (FORS) technology developed by Koninklijke Philips N.V.

One example of a suitable mechanism for constructing a model of the internal anatomy of the subject based on the location measurements is disclosed by the European Patent Application having the publication number EP 3568068 A1.

There is an ongoing desire to improve the accuracy of anatomical models constructed from location data and to provide contextual information for improving an understanding of constructed anatomical models.

### SUMMARY OF THE INVENTION

The invention is defined by the claims.

According to examples in accordance with an aspect of the invention, there is provided an anatomical model registration system for registering an anatomical model of an anatomical cavity, generated using location measurements provided using a location sensor carried by an interventional device and representing different positions of the interventional device within the anatomical cavity, to a reference anatomical model of the anatomical cavity.

The anatomical model registration system comprises: an input interface configured to: receive location data comprising a sequence of location measurements, wherein each location measurement in the sequence represents a temporally later position of the interventional device than any other location measurement earlier in the sequence; and receive the reference anatomical model for the anatomical cavity.

The anatomical model registration system also comprises a data processor configured to: process the location data to generate an anatomical model of the anatomical cavity; use the sequence of location measurements to define a path taken by the interventional device; and register the generated anatomical model to the reference anatomical model using the defined path taken by the interventional device.

The present disclosure proposes an approach for registering an anatomical model generating from location data (of an interventional device) to a reference anatomical model, e.g., an anatomical model generated or derived from imaging data of the anatomical cavity.

A sequence of location measurements are used to define or determine a path taken by the interventional device within the anatomical cavity. A path is a route or trajectory taken by the interventional device, and can be represented by a line (e.g., a straight line, an arc, a parabola or other parametric shape) within a Euclidean space system. Other suitable approaches for defining a path would be apparent to the skilled person.

This path is then used to register the generated anatomical model (produce using the location data) to the reference anatomical model. Is it recognized that a path taken by an interventional device should match or correspond to a potential path or route available to the interventional device defined by the reference anatomical model or to the shape of a particular part of the anatomical cavity represented by the reference anatomical model.

The anatomical model registration system may be configured to perform at least the step of registering the generated anatomical model to the reference anatomical model whilst the interventional device is within the anatomical cavity. This can provide useful information for guiding or aiding in an imaging process. In particular, registering the reference anatomical model to the generated anatomical model can aid in, for example, automatic labelling or annotating of the generated anatomical model and/or guiding further investigation of the anatomical cavity using the interventional device to improve the accuracy or completeness of the anatomical model.

Each location measurement may be an electrical response of a device electrode carried by the interventional device to crossing electrical fields induced within the anatomical cavity. Other suitable examples of a location measurement include a magnetic response of a magnetic sensor to one or more magnetic fields induced with the anatomical cavity. Yet another suitable example is a response of an ultrasound sensor to one or more ultrasound waves emitted into the anatomical cavity.

Thus, each location measurement may be an intra-body measurement that represents a measurement taken within the anatomical cavity of the subject.

Yet another suitable example is the response of an external sensor to a magnetic impulse emitted by the location sensor. Yet another suitable example is the response of an external sensor to an ultrasound emitted by the location sensor. These provide examples of location measurements that are taken externally to the anatomical cavity of the subject, but responsive to an emission from a sensor carried by an interventional device within the anatomical cavity of the subject.

In the context of the present invention, registering two models means to spatially register the two models, e.g., determine a scaling and/or rotation to be applied to one or both models such that they geometrically align with one another. This effectively places both models in a same co-ordinate system, e.g., at a same position, scaling and/or rotation in the co-ordinate system.

The system may further comprise an output interface configured to control a user interface to provide a visual representation of the generated anatomical model and the reference anatomical model, wherein the visual representation is responsive to the registration of the generated anatomical model and the reference anatomical model.

This approach helps to guide an operator of the interventional device to areas of the anatomical cavity that have not yet been reached by the interventional device, i.e., areas for which no location measurements have yet been generated. This approach thereby provides an operator with useful information for improving the imaging of the anatomical cavity.

In some examples, the system may comprise an output interface configured to control a user interface to provide a visual representation of the defined path taken by the interventional device. This approach may, for instance, provide the visual representation relative or with respect to the visual representation of the generated anatomical model and/or reference anatomical model. This aids the operator in understanding their current progress.

The anatomical model registration system may be configured wherein: the data processor is further configured to process the reference anatomical model to identify a plurality of potential paths for an interventional device; and the data processor is configured to register the generated anatomical model to the reference anatomical model by processing the defined path and the plurality of potential paths.

In this way, potential paths through the reference anatomical model can be used to register the defined path (and thereby the anatomical model) to the reference anatomical model.

The data processor may be configured to register the generated anatomical model to the reference anatomical model by comparing the defined path to each potential path to determine which of the plurality of potential paths best matches the defined path; and registering the generated anatomical model to the reference anatomical model using the defined path and the best matching potential path.

In some examples, the data processor is configured to register the generated anatomical model to the reference anatomical model by: defining, for each potential path, an identity of the part of the anatomical cavity through which the potential path passes; processing the defined path to identify the part of the anatomical cavity through which the defined path passes; identifying which of the plurality of potential paths identifies the same part of the anatomical cavity as the defined path; and registering the generated anatomical model to the reference anatomical model using the defined path and the identified potential path.

In some examples, the anatomical cavity is a cavity in a cardiac system of a subject and the plurality of potential paths contain at least: a path through a vena cava; a path through a coronary sinus; and a path through an aortic arch.

In some examples, the reference anatomical model is an anatomical model derived from imaging data of the anatomical cavity. The imaging data may comprise magnetic resonance imaging data; computed tomography imaging data; or 3D ultrasound imaging data.

In other examples, the reference anatomical model is an anatomical atlas representing a population-derived anatomical model of the anatomical cavity. This approaches allows for automated labelling of the anatomical model, e.g., using labels of the spatially registered anatomical atlas.

The data processor may be configured to use the sequence of location measurements to define a path taken by the interventional device by determining a line of best fit through the sequence of location measurements, the line of best fit representing the path taken by the interventional device. This provides an effective and efficient mechanism for determining a path or line representing a path taken by the interventional device. The line of best fit may be a line definable using a first, second or third order polynomial.

In some examples, the input interface is configured to receive further location measurements, each further location measurement representing a temporally later position of the interventional device than any location measurement in the sequence of location measurements; and the data processor is configured to update the anatomical model of the anatomical cavity using the further location measurements. There is a particular advantage of the proposed approach in the early stages of producing an anatomical model, e.g., when there is insufficient location data to produce a highly accurate anatomical model for rendering and display. This is because the registered reference model can give contextual information on the generated anatomical model, to aid an operators understanding of the anatomical model being produced and/or the location of the interventional device with respect to the anatomical model.

In preferred examples, the generated anatomical model is a polygon mesh, and optionally the reference anatomical model is a polygon mesh. A polygon mesh is particularly advantageous, as they contain less information than the complete location data whilst providing useful clinical information for an operator.

There is also proposed a computer-implemented method for registering an anatomical model of an anatomical cavity, generated using location measurements provided by a location sensor carried by an interventional device and representing different positions of the interventional device within the anatomical cavity, to a reference anatomical model of the anatomical cavity.

The computer-implemented method comprises: receiving location data comprising a sequence of location measurements, wherein each location measurement in the sequence represents a temporally later position of the interventional device than any other location measurement earlier in the sequence; receiving the reference anatomical model for the anatomical cavity; processing the location data to generate an anatomical model of the anatomical cavity; using the sequence of location measurements to define a path taken by the interventional device; and registering the generated anatomical model to the reference anatomical model using the defined path taken by the interventional device.

The method may be configured to comprise the steps taken by any herein described system, and vice versa.

There is also proposed a computer program product comprising computer program code means which, when executed on a computing device having a processing system, cause the processing system to perform all of the steps of any herein described method.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates a system for analyzing or investigating the anatomical cavity of a subject;
Fig. 2 illustrates a process for reconstructing an anatomical model;
Fig. 3 conceptually illustrates the proposed approach;
Fig. 4 illustrates a computer-implemented method according to an embodiment;
Fig. 5 illustrates a registration process for use in an embodiment;
Fig. 6 illustrates an anatomical model registration system according to an embodiment; and
Fig. 7 illustrates a more detailed example of the anatomical model registration system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for registering an anatomical model, generated from location measurements, to a reference anatomical model. A path taken by an interventional device (for which the location measurements are obtained) is determined. The determined path is used to register the anatomical model to the reference anatomical model.

The present invention is based on the realization that a path taken by an interventional device is defined by the shape of the region through which the interventional device passes. Thus, is it possible to identify the part of the reference model representing the region through which an interventional device is expected to have moved based on its path. In particular, it is possible to identify a reference path (defined with respect to the reference anatomical model) that best matches the actual path (defined with respect to the generated anatomical model) taken by the interventional device. This makes it possible to register the two anatomical models together without needing to attempt to register the overall model.

The proposed technique thereby facilitates more efficient registration of a generated anatomical model to a reference anatomical mode, and at an earlier stage in the process of generating the anatomical model (e.g., before the anatomical model is generated to a high degree of accuracy).

Embodiments can be employed in any scenario in which location data for an interventional device is available and can be used to generate an anatomical model, e.g., during/following interventions.

In the context of the present invention, a "line" is considered to include both straight lines and curves (i.e., curved lines). A curve may be a polynomial curve, e.g., include one or more bends or maxima/minima, and be parametrized using a polynomial function of order N, where N is any real positive integer (i.e. N is in the set R+).

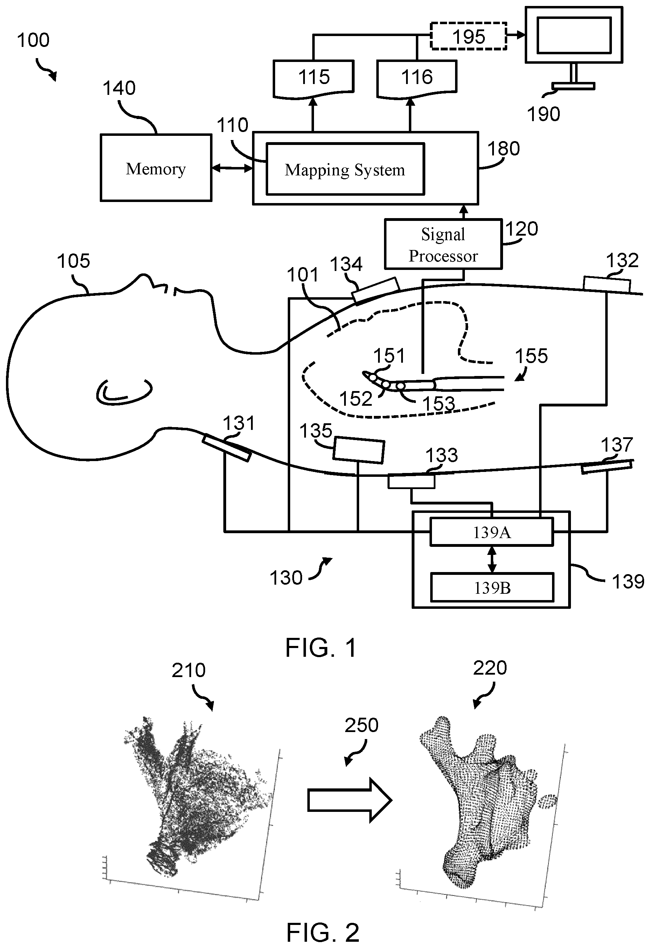

Fig. 1 illustrates a system 100 for analyzing or investigating an anatomical cavity 101 of a subject 105.

The anatomical cavity 101 may, for instance, be an anatomical cavity within any suitable anatomical element of the subject 105, such as the heart, liver, kidney, lungs etc. or parts thereof. Preferably, the cavity 101 is an atrium or ventricle of the heart.

An interventional device 150 of the system 100 carries one or more location sensors 151, 152, 153. Each location sensor 151, 152, 153 can be used to define or determine location measurements that each represent a position of the interventional device 150 within the anatomical cavity 101.

The interventional device 150 may be or comprise a catheter, such as an electrophysiological mapping catheter or an ablation catheter, although other devices or catheters may be used.

In the illustrated approach, each location sensor 151, 152, 153 is a field-sensitive sensor that produces a location measurement that differs depending upon the position of said field-sensitive sensor within one or more crossing electromagnetic fields induced within the subject, and therefore depending upon the position of said field-sensitive sensor within the anatomical cavity 101. The location measurement may comprise one or more location data points, each location data point being a response of the field-sensitive sensor to a particular electromagnetic field.

In other approaches, at least one location sensor 151, 152, 153 is a signal emitter, e.g., for emitting an electrical or electromagnetic signal. An array of one or more external sensors (positioned externally to the subject) can generate a location measurement responsive to the emitted signal by the signal emitter. Such a location measurement would also change responsive to the position of said signal emitter within the anatomical cavity 101. Similarly, each external sensor may provide a location data point that collectively form the location measurement, each location data point being a response of said external sensor to the emitted signal by the signal emitter.

In techniques where each location sensor is a field-sensitive sensor, any raw signals at location sensor(s) 151, 152, 153 may be filtered, sampled and/or digitized using a signal processor 120 of the system 100 to produce the location measurement. In particular, the signal processor 120 may (electrically) connect to the location sensor(s) 151, 152, 153 and process the raw signals at the location sensor(s) to produce a location measurement for digital processing by a processing system or other similar device. The signal processor 120 may therefore comprise appropriate circuitry for performing such functions, e.g., filtering circuitry, sampling circuitry and/or digitizing circuitry (e.g., an analogue-to-digital converter).

One example of a suitable location measurement is a set of voltages induced at the location sensor by external electromagnetic fields induced in the subject. Thus, in some examples, the location sensor is an electrode ("device electrode") at which a set of voltages, containing a voltage for each electromagnetic field, differs depending upon the position of said location sensor with respect to the electromagnetic fields. In this scenario, the signal processor 120 may be able to sample and/or filter a set of voltages at each location sensor to produce the location measurement.

Another example of a suitable location measurement is a magnetic response at the location sensor by electromagnetic fields induced in the subject. Thus, in some examples, the location sensor is a magnetic sensor (such as a Hall-effect sensor or a set of coils) at which a magnetic response of the magnetic sensor differs depending upon the position of said sensor with respect to the electromagnetic field. In this scenario, the signal processor 120 may be able to sample and/or filter the voltage(s) (or other electrical response) produced by each magnetic sensor to produce the location measurement.

The signal processor 120 may be appropriately configured for producing the sets of measurements, e.g., to sample and/or filter the raw sensor signals produced by the field-sensitive sensors for digital processing.

In techniques where each location sensor 151, 152, 153 is a field-sensitive sensor, the system 100 may comprise an electromagnetic field generating arrangement 130 for generating the electromagnetic field(s). An electromagnetic field generating arrangement 130 comprises a set of field generators 131, 132, 133, 134, 135, 137 operably connected to a field controller 139.

The arrangement 130 may be configured to generate crossing electromagnetic fields within at least a part of subject 105 using the set of field generators positioned externally with respect to the subject 105. The field generators may include electrodes (for generating electric fields) or solenoids (for generating magnetic fields). Other suitable examples will be apparent to the skilled person.

The field controller 139 is configured to control characteristics (e.g., voltage and/or current) of the electric signals provided to each field generator 131, 132, 133, 134, 135, 137 to thereby generate the crossing fields. For example, the arrangement may generate alternating electromagnetic fields as the crossing electromagnetic fields.

The field controller 139 may comprise a suitable signal generator 139A for generating and transmitting signals to each field generator 131, 132, 133, 134, 135, 137. The operation of the signal generator 139A may be controlled by a field controller processing system 139B of the field controller 139 (e.g., which may form part of any other processing circuitry of the system 100).

To distinguish between different electromagnetic fields (in the location measurement taken at a field-sensitive sensor), each electromagnetic field may be generated with a different and distinguishable frequency and/or temporal code. A location data point produced by the location sensor may be an average or instantaneous measurement or response at the location sensor to a particular frequency and/or temporal code, corresponding to the frequency and/or temporal code of the corresponding electromagnetic field. The combination of the location data points can provide the location measurement.

The field controller 139 may be configured to control the electromagnetic fields to operate in the frequency range of 10 kHz to 1 MHz, preferably in the range of 10-100 kHz. This frequency range is particularly useful for ensuring penetration of a subject, whilst providing a frequency range that attenuates in human tissue without significant damage/injury to the tissue.

The reference sensor (e.g., 137) can serve, if required for the type of measurement, as a reference, e.g., a measurement reference, for all measurements taken by sensors, e.g., as a reference for the response of the device sensors.

It will be apparent that the location measurement will change as the relative position of the location sensor(s) move(s) about the subject. Such movement may be caused by a physician deliberately roving or moving the location sensor within the cavity 101, to collect location measurements at different locations within the cavity 101.

The principle of generating location measurements thereby facilitates tracking of the relative position of the interventional device 150 using a mapping system 110 that monitors the measurements of any location sensor. The measurements are obtained or contained within a measurement coordinate system, sometimes referred to as a "measurement space".

In approaches where each location measurement comprises one or more location data points, and each location data point is a response of the field-sensitive sensor to a particular electromagnetic field, it may be necessary to map the location measurement to a spatial position in a spatial co-ordinate system, sometimes referred to as "R-space". This need is due to the inherent inhomogeneity of the electromagnetic field(s) in the subject, meaning that the scale between different location measurements may not accurately represent a true distance. A spatial co-ordinate system is one that represents a true spatial relationship (e.g., proportionally scaled to geometric/physical distances) between elements in the spatial co-ordinate system.

Mapping can be performed using a suitable (location) measurement-to-position or mapping function, sometimes referred to as an "M2R function". If the location measurement is a set of one or more voltages, this function can be labelled a voltage-to-position ("V2R") function. The mapping function therefore converts a location measurement in a measurement coordinate system into a location or position (e.g., set of co-ordinates) in a spatial co-ordinate system. This mapping function will therefore determine the relative recorded position(s) of the sensors, and therefore of the interventional device, in a spatial co-ordinate system (e.g., compared to a measurement co-ordinate system).

Approaches for generating such a mapping function are well established in the art. Example processes are disclosed by the European Patent Applications EP 0775466 A2, EP 3568068 A1 and EP 3607879 A1.

Generating a mapping function typically makes use of known inter-sensor distances to calibrate for the inhomogeneity in the electromagnetic fields. Thus, it is typically necessary to know the distance between each of the location sensors. Historically, these have been provided by the manufacturer or operator of the interventional device.

It will be appreciated that mapping a location measurement to a spatial position is an optional step. Indeed, in some approaches, a location measurement may already be sufficient to accurately represent the "true" location of the location sensor in space.

Once a number of locations of the interventional device and/or one or more sensors on the interventional device in the spatial co-ordinate system have been established, it is possible to construct an anatomical model 115 of the anatomical cavity 101. This process may be performed by the system 100, or a subsystem thereof such as a mapping system 110.

A brief description of how to generate an anatomical model is provided for improved contextual understanding. This description also aids to emphasize a particular use-case scenario for data derived from a measurement-to-position function, and therefore the clinical benefit(s) and advantage(s) of a more accurate measurement-to-position function.

Broadly, the mapping system 110 may build an anatomical model 115 of an anatomical cavity 101 (e.g., a chamber, vessel or void) within a subject by processing a cloud of points (i.e., location data). Each point represents a location or position of the interventional device or sensor in the spatial co-ordinate system, and could therefore be represented as a set of data, e.g., a set of co-ordinates.

More specifically, a reconstruction algorithm is used to generate an anatomical model of the (investigated part of the) anatomical cavity. The anatomical model may, for example, be a 3D (polygon) mesh or surface that depicts or models the (known bounds of the) anatomical cavity. In the present disclosure, the anatomical model preferably comprises a 3D mesh defined by a plurality of vertices, each vertex being associated with a different location in the predefined co-ordinate system. The 3D mesh is therefore defined in the same co-ordinate system as the determined locations of the field-sensitive sensor(s). The 3D mesh may, for instance, be defined as sets of co-ordinates defining vertices of the 3D mesh.

The process of reconstructing an anatomical model from a point cloud is conceptually illustrated in Fig. 2, which demonstrates a process 250 in which a cloud of points 210 ("point cloud") is transformed into an anatomical model 220. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

Thus, it is possible to generate an anatomical model 115 of the anatomical cavity 101 using location data comprising location measurements obtained using the location sensor(s).

Referring to Fig. 1, the anatomical model may be stored, for instance, in a memory 140 or storage unit. Of course, the anatomical model may be output for further processing and/or to a user interface 190, such as a screen or display, configured to provide a visual representation of the anatomical model.

Thus, the system 100 may comprise a user interface 190 configured to provide a visual representation of the anatomical model. In some examples, a display processor 195 may be configured to receive the anatomical model 115 from the mapping system 110 and render the anatomical model to generate display data that can be received and processed by the user interface 190 for providing a visual representation of the anatomical model.

More particularly, once a 3D mesh has been generated, then a visual representation of the 3D mesh can be provided. Providing a visual representation of the 3D mesh may comprise, for example, rendering a reconstruction of the surface of the (known) anatomical cavity. The visual representation may be provided to a user with an appropriate output device such as a display device. The system 100 may include the appropriate output terminals connectable to or connected to the output device according to known principles.

In one method, the steps of gathering location measurements and reconstructing an anatomical model using the gathered location measurements can be repeated until an acceptable anatomical model has been obtained.

Once an anatomical model has been generated, it is possible to monitor the location of the location sensor(s) and/or interventional device with respect to the anatomical model. For example, both the model and the tracking of location of the device may have the shared spatial coordinate system. The system 100 or the mapping system 110 may be accordingly configured to determine a location of the location sensor(s) and/or the interventional device. This may comprise identifying or determining one or more (possibly new) locations 116 of each location sensor and/or interventional device. This may include sampling additional location measurements overtime and mapping such additional sets to respective additional locations in R-space using the mapping function. A model of the interventional device comprising the known locations of the location sensors on the device may be used to generate a location of at least a part of (e.g. preferably the part comprising one or more of the sensors) the interventional device.

The anatomical model in combination with monitored additional (new) locations may be used, for instance, to provide a visual representation of the known bounds of the anatomical cavity (represented by the anatomical model) and the relative position of the interventional device and/or its sensor(s) with respect to the anatomical cavity. This provides useful clinical information to an operator of the interventional device, e.g., to track the progress (e.g. location and/or orientation) of the interventional device and/or view the shape/structure/size of the anatomical cavity in which the interventional device is to be moved or is moving.

The present disclosure provides a technique for registering the anatomical model generated using the location data with a reference anatomical model. The reference anatomical model represents a same type of anatomical cavity as that represented by the anatomical model. For instance, if the anatomical model represents a cavity in the heart, then the reference anatomical model will also represent a cavity in the heart.

The reference anatomical model may, for instance, be an anatomical model that was produced from non-invasively obtained imaging data of the anatomical cavity of the subject (e.g., MRI data, CT data or 3D ultrasound data) or be an anatomical model represent a population average anatomical model (e.g., produced using anatomical models derived from location data obtained for a plurality of other subjects).

One approach for producing an anatomical model from imaging data of the anatomical cavity is to use a segmentation procedure. Segmentation procedures are able to automatically generate a polygon mesh or other model of an imaged region, such as the anatomical cavity.

In some examples, the reference anatomical model may be an anatomical atlas representing a population-derived anatomical model of the anatomical cavity. Such a reference anatomical model may be associated with a set of labels or annotations, that identify or provide identifying information about different regions of anatomical model (e.g., identifying or labelling the part of the anatomical cavity represented by the region of the anatomical model). Such annotations may, for instance, be provided by a skilled clinician labelling the reference anatomical model.

With reference to Fig. 1, the present disclosure thereby provides an anatomical model registration system 180 for registering the anatomical model with a reference anatomical model (e.g., retrieved from a memory or storage unit 140). In the illustrated example, the anatomical registration system 180 comprises the mapping system 110.

The present disclose also proposes a system 100 comprising the anatomical model registration system 180 and the user interface 190. Of course, the system 100 may further comprise the interventional device 150, the signal processor 120, and/or the memory 140. The system may, where the interventional device 150 carries one or more field-sensitive sensors as the location sensor(s), comprise the electromagnetic field generating arrangement 130. In some examples, the system 100, where the interventional device carries a signal emitter as the location sensor(s), comprises a sensor array (not shown) for monitoring the emitted signal and generated the location measurement(s).

If present, the user interface 190 may be configured to provide a visual representation of the anatomical model and the reference anatomical mode. The visual representation of the anatomical models are preferably spatially registered with respect to one another.

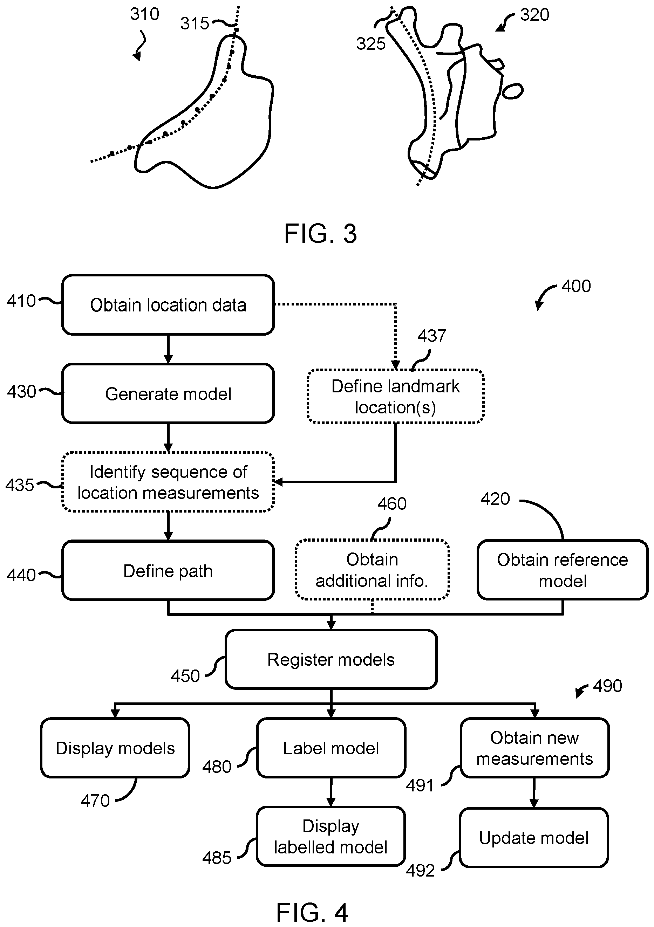

The principle of the herein proposed approach is illustrated in Fig. 3.

Fig. 3 illustrates the anatomical model 310 (generated from the location data) and the reference anatomical model 320. The anatomical model 310 has been generated from insufficient location data, such that it is not yet able to accurately represent all the features of the anatomical cavity with a high degree of accuracy.

Initially, the spatial registration (e.g., the rotational and/or scaling relationship) between the two models 310, 320 is unknown.

It has been recognized that it is possible to use a sequence of location measurements (illustrated as small circles) to define a path 315 or route taken by the interventional device as it moves through the anatomical cavity. The spatial relationship between the path 315 and the anatomical model 310 is known, as the anatomical model is constructed from the location data including a sequence of location measurements used to produce/define the path 315.

This path 315 or route is indicative of the surrounding tissue or structure of the anatomical cavity, as the bounds of the anatomical cavity will restrict a possible path for the interventional device. An identified path 315 taken by an interventional device can therefore be used to identify, in the reference anatomical model 320, the most likely region of the reference anatomical model through which the interventional device is moving. Thus, a reference path 325 can be identified, being a corresponding path or route in the reference anatomical model that matches or corresponds to the identified path 315.

If the location of the path 315 in the anatomical model 310 and the most likely corresponding location for a reference path 325 in the reference anatomical model 320 can be identified, it is possible to spatially register the anatomical model to the reference anatomical model. This is because the spatial relationship between the path and the anatomical model is known, as is the spatial relationship between the most likely position for the path in the reference anatomical model and the reference anatomical model itself is known. Thus, it is possible to rotate and/or scale one of the anatomical models (and thereby the corresponding path) until the two paths align or match one another spatially.

In the context of the present invention, a path can be considered to be a line, such as a straight line or curve, which identifies a route taken by the interventional device or a location sensor.

Fig. 4 illustrates a computer-implemented method 400 according to an embodiment of the invention. The method 400 may be carried out by the anatomical model registration system previously mentioned.

More particularly, the method 400 is for registering an anatomical model of an anatomical cavity, generated using location measurements provided by a location sensor carried by an interventional device and representing different positions of the interventional device within the anatomical cavity, to a reference anatomical model of the anatomical cavity.

The method 400 comprises a step 410 of receiving location data comprising a sequence of location measurements, wherein each location measurement in the sequence represents a temporally later position of the interventional device than any other location measurement earlier in the sequence. The location data may comprise additional location measurements (i.e., location measurements that do not contribute to the sequence of location measurements).

A temporally later position is a position that the interventional device reaches after reaching a temporally earlier position. Thus, the sequence is ordered by time of acquisition or sampling of each location measurement.

Step 410 merely comprises a passive process of obtaining or receiving the location measurements, e.g., from a memory, storage device or other processing system. Step 410 does not need to comprise the active process of actively determining the location measurements. The above-described techniques are provided for the sake of improved contextual understanding. Of course, some systems may perform this active process, but it is not essential for performing method 400. Thus, process 400 is a data processing method for processing previously obtained data.

The method 400 also comprises a step 420 of receiving the reference anatomical model for the anatomical cavity.

The reference anatomical model may be predetermined, e.g., a known reference anatomical model. This is advantageous if the purpose of the anatomical model registration system is known in advance (e.g., if the system is designed for a particular cavity).

In some examples, one of a plurality of potential reference anatomical models is selected as the reference anatomical model. The selection may be performed manually, e.g., responsive to a user input. Thus, in some examples, step 420 comprises receiving a user input identifying a potential reference model and obtaining the identified potential reference model as the reference model.

Steps 410 and 420 may be performed by an input interface of the anatomical model registration system. The sequence of location measurements may be obtained from the signal processor 120 or a memory 140 or storage unit. The reference anatomical model may be retrieved from the memory 140 or storage unit.

The method 400 also comprises a step 430 of processing the location data to generate an anatomical model of the anatomical cavity. Step 430 may be performed by a data processor of the anatomical model registration system. In particular, step 430 may be performed by the mapping system 110, which may form part of the data processor.

Approaches for processing location data to generate an anatomical model have been previously described. Preferably, the anatomical model is a polygon mesh, for which mechanisms for generating have also been previously described.

The method 400 also comprises a step 440 of using the sequence of location measurements to define a path taken by the interventional device. The path may be defined in a measurement space or, if mapping is used/available, in the R-space.

This can be performed, for instance, by determining a trendline or line of best fit through the sequence of location measurements. As each location measurement can effectively represent a co-ordinate in a measurement space, or a co-ordinate in an R-space, it is possible to determine a line of best fit through the location measurements.

If generated, the line of best fit may be defined in the measurement space or the R-space. If defined in the measurement space, it is possible to map the line of best fit to the R-space using the mapping function.

The line of best fit may be a line definable using a first, second or third order polynomial. It has been recognized that these order polynomials are adequate to suitably describe or define a path taken by an interventional device within an anatomical cavity for the purposes of registering anatomical models. Higher order models may be used, but require increased processing power/time to determine.

Approaches for determining a line of best fit are well known in the field of statistical analysis, and could be applied to the present invention. Generally, a (linear or non-linear) regression analysis is performed in order to determine a line of best fit. For instance, computing a second order parametric fit model can be performed using a standard linear algebra poly-fit function.

Another approach for performing step 440 is to simple determine a line that connects each location measurement (or mapped location measurement in R-space) to one another, i.e. determine a polyline where each location measurement represents a vertex. This connecting line would be connected in order of time of acquisition of each location measurement.

In some examples, step 440 is preceded by a step 435 of processing the location data to identify a suitable sequence of location measurements for defining the path. The location data comprises a plurality of location measurements, captured at different points in time.

In one example, step 435 comprises processing the location data to identify a sequence of location measurements that together form an elongated shape, e.g., a shape that could be processed to generate a line. An elongate shape for a sequence is one in which the location measurements (in time order) generally move away from one another.

Thus, step 435 may comprise processing the location data to identify a sequence of location measurements that form an elongated path. If step 435 is unable to identify such a sequence of location measurements, then the method may revert back to step 410 to obtain additional location data.

In some embodiments, step 435 makes use of landmark data in order to identify the limits of the path, e.g., the start and end locations of the path or the start and end of the sequence of location measurements.

Thus, the method 400 may comprise a step 437 of defining or identifying the location of one or more landmarks with respect to the anatomical model. Step 435 may comprise using the identified location(s) to identify the start and/or end of the sequence of location measurements.

The landmark(s) for whose locations are defined in step 437 may be landmarks for a predetermined path, e.g., landmarks identifying the entry/exit to a particular lumen in the anatomical cavity or landmarks identifying when the device is in a path. For instance, if the path is to be a path through the vena cava, then the landmarks(s) may identify a location of an entry and/or exit to the vena cava. Step 435 may comprise identifying at least the location measurement(s) associated with the location(s) at or closest to the landmark location(s) and setting the start/end of the sequence accordingly.

It is possible to automatically detect when the interventional device is at a landmark location responsive to electrical signals in the vicinity of the location sensor(s) caused by electrical activity of the anatomical element/structures. Such electrical signals lie in a set wavelength band (e.g., from 0/0.5/5 Hz to 100/150 Hz), and can therefore be distinguished from any electrical signals induced by electromagnetic fields (if this approach is used to generate the location measurements).

As an example, there is a step amplitude change in such electrical signals when entering the pulmonary vein. In particular, when entering the pulmonary veins, a decrease in the amplitude of the electrical signal(s) can be seen. This feature can be used to detect any PV locations, each being the location of any pulmonary vein through which the interventional device travels or nears.

Other anatomical elements or landmarks have similar identifying characteristics of the electrical signal. For instance, there are similar changes in amplitude of the electrical signals with increased distance along the inferior vena cava and superior vena cava.

The electrical signals in this set wavelength band may be acquired at a same time as obtaining a location measurement. It is therefore possible to associate landmarks (as determined from the relevant electrical signals) with a particular location measurement, thereby defining a landmark location. The landmark location(s), i.e., the location measurements associated with an identified landmark, can be used in step 435 to define the sequence of location measurements.

Thus, in some embodiments, the interventional device carries one or more electrodes for detecting electrical signals in the vicinity of the interventional device. In such embodiments, the method may comprise receiving and processing electrical signals to identify one or more landmarks indicative of the path taken by the interventional device. A step of processing 435 the location data to identify a sequence of location measurements may comprise identifying the location measurement(s) associated with any identified landmark or landmark locations. The identified location measurement(s) may then be used to identify the sequence of location measurements.

The method 400 further comprises a step 450 of registering the generated anatomical model to the reference anatomical model using the defined path taken by the interventional device.

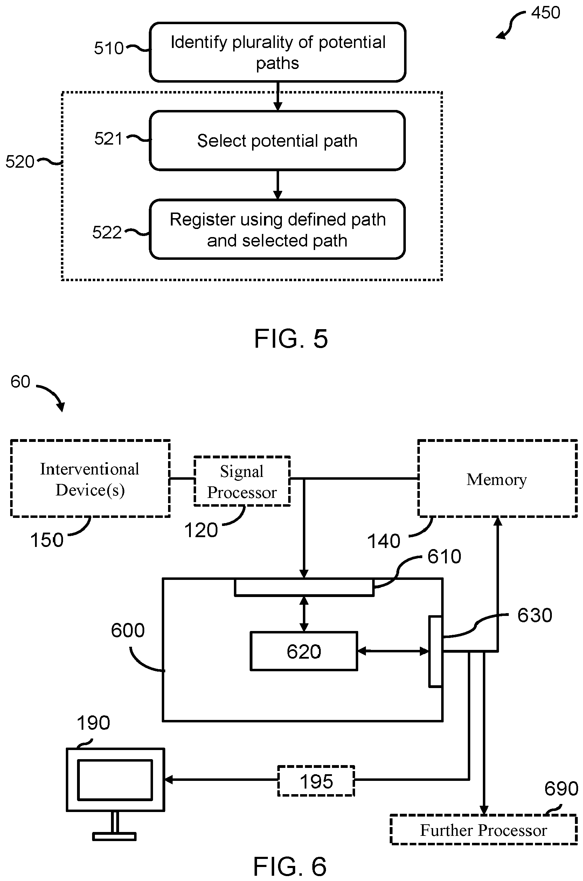

Fig. 5 is a flowchart illustrating one approach for registering the generated anatomical model to the reference anatomical model using the defined path, i.e., performing step 450. Thus, Fig. 5 illustrates a registration process for use in an embodiment.

Step 450 may comprise a step 510 of processing the reference anatomical model to identify a plurality of potential paths for an interventional device. A potential path identifies a potential path or route that an interventional device may take when moving through the anatomical cavity represented by the anatomical model. It will be apparent that a potential path is a potential reference path, i.e., a potential path through the reference anatomical model that may match or correspond to the defined path.

The plurality of potential paths may be predetermined. This can allow, for instance, known paths or routes through the anatomical cavity to be identified. For instance, if the anatomical cavity is a cavity in a cardiac system of a subject, then the plurality of potential paths contain at least: a path through a vena cava; a path through a coronary sinus and/or a path through a pulmonary vein.

Step 450 may then perform a process 520 of registering the generated anatomical model to the reference anatomical model by processing the defined path and the plurality of potential paths.

Process 520 can be performed by first carrying out a step 521 of selecting one of the potential paths based on the defined path. The selected potential path can be simply referred to as the "reference path".

In one example, step 521 is performed by comparing the defined path to each potential path to determine which of the plurality of potential paths best matches the defined path and selecting the best matching potential path as the selected path. The best matching potential path may be the path that differs the least (e.g., in terms of shape or structure) from the defined path. This can be achieved through shape-matching algorithms, which are well known in the art. One example (for a polyline) is a turning function. One example (for a trend line or line of best fit) is to determine an area under a curve

In another example, step 521 is performed by first defining, for each potential path, an identity of the part of the anatomical cavity through which the potential path passes. The defining of each potential path may be performed in advance, e.g., by a suitably trained clinician or automatically by a segmentation and classification process. Different potential paths will go through different parts of the anatomical cavity. The defined path is then processed to identify the part of the anatomical cavity through which the defined path passes. This recognizes that the shape of a path can effectively define which part of the anatomical cavity the path is passing through. This can be performed using a suitably trained classification function or the like (e.g., a machine-learning method), or through knowledge of known characteristics of a path through certain anatomical features. Step 521 may then select one of the plurality of potential paths that identifies or passes through the same part of the anatomical cavity as the defined path.

In this way, step 521 can effectively select one of the potential paths as a reference path based on a measure of similarity with the defined path or a determination of which potential path (i.e., reference path) passes through a same region/area of the anatomical cavity as the defined path. Other approaches would be apparent to the skilled person.

Thus, step 521 effectively associates or matches the defined path with a corresponding reference path in the reference model.

Process 520 may then perform a step 522 of registering the generated anatomical model to the reference anatomical model using the defined path and the selected potential path.

Step 522 can, for instance, be performed by defining a required rotation and/or scaling factor to be applied to the anatomical model and/or the reference anatomical model such that the potential path and the selected potential path are best aligned spatially or best registered with respect to one another. This approach will simultaneously register the two anatomical models together.

Turning back to Fig. 4, it is possible to use additional information, as well as the defined path, in registering the anatomical model 310 to the reference anatomical model 320.

For instance, it is possible to automatically detect when the interventional device is near certain anatomical elements or structures responsive to electrical signals in the vicinity of the location sensor(s) caused by electrical activity of the anatomical element/structures. Approaches for performing this process have been previously described.

Accordingly, method 400 may further comprise a step 460 of obtaining additional information, and step 450 may be modified to further use the additional information to register the anatomical model to the reference anatomical model.

In some examples, the additional information comprises obtaining one or more electrical measurement sampled at the location sensor in a first wavelength band. The first wavelength band may be from X Hz to Y Hz, where X = 0, 0.05 or 0.5Hz and Y = 100 or 150.

The electrical measurement(s) can be used to identify one or more anatomical landmarks in the anatomical model, e.g., a location of a particular anatomical element. These anatomical landmarks can be used to aid in the registration of the anatomical model to the reference anatomical model (e.g., by positioning the anatomical landmark(s) at positions of reference anatomical landmark(s) on the anatomical model).

The method 400 may also comprise a step 470 of controlling a user interface to provide a visual representation of the generated anatomical model and the reference anatomical model. The visual representation is responsive to the registration of the generated anatomical model and the reference anatomical model. Thus, step 470 effectively comprises displaying the generated anatomical model and the reference anatomical model (at a user interface).

Approaches for performing step 470 will be readily apparent to the skilled person, and may comprise rendering the anatomical model and the reference anatomical model to generate display data, which is usable by the user interface to provide a visual representation (e.g., as a screen or similar device).

For instance, the visual representation may visually represent the generated anatomical model and the reference anatomical model at a same scale and rotation (i.e., spatially registered with respect to one another).

If performed, step 470 may be carried out by an output interface of the anatomical model registration system.

As previously explained, the reference anatomical model may be an anatomical atlas representing a population-derived anatomical model of the anatomical cavity. Such a reference anatomical model may be associated with a set of labels or annotations, which identify or provide identifying information about different regions of anatomical model (e.g., identifying or labelling the part of the anatomical cavity represented by the region of the anatomical model).

In such examples, the method 400 may also comprise a step 480 of labelling the defined anatomical model (i.e., the anatomical model produced from the location data) using the label(s)/annotation(s) of the reference anatomical model. In particular, once the defined anatomical model and the reference anatomical model have been spatially registered, it is possible to directly map labels/annotations of the reference anatomical model to the defined anatomical model, e.g., to label parts of the defined anatomical model with the label(s) of the spatially corresponding parts of the reference anatomical model.

This provides a mechanism for automatic labelling of the anatomical model.

The method 400 may further comprise a step 485 of controlling a user interface to provide a visual representation of the labelled anatomical model. This provides a clinician with additional useful information about the anatomical model that was not previously available.

If step 470 is performed, then step 485 may form part of step 470. Of course, step 485 may be performed independently.

More generally, the method 400 may comprise a step of generating or defining output data. The output data may be used to control a user interface to provide a visual representation of the output data (e.g., as exemplified by steps 470 and 485). Alternatively, the output data may be output to a further processing device for further processing and/or analysis.

As one example, the output data comprises the registered anatomical model and reference anatomical model itself. This is exemplified by step 470 that controls a user interface to provide a visual representation of such output data.

As another example, the output data comprises registration information that defines how the anatomical model and reference anatomical model are spatially related (e.g., a rotation and/or scaling required to spatially register the two models.

As yet another example, the output data may comprise a labelled anatomical model. Production of such output data is exemplified by step 480.

In some examples, the output data comprises the defined path. Thus, it is possible to provide a visual representation of the defined path, e.g., with respect to the generated model and/or registered models. The location of the defined path with respect to the generated path is known, as previously explained.

The method 400 may further comprise an anatomical model update process 490.

This process 490 comprises a step 491 of receiving further location measurements, each further location measurement representing a temporally later position of the interventional device than any location measurement in the sequence of location measurements. The further location measurements may then be considered to form part of the location data.

The process 490 also comprises a step 492 of updating the anatomical model of the anatomical cavity using the further location measurements. Step 492 may, for instance, comprise regenerating the anatomical model using the location data (including both the sequence of location measurements and the further location measurement).

This approach recognizes that there is a particular advantage of the proposed approach in the early stages of producing an anatomical model, e.g., when there is insufficient location data to produce a highly accurate anatomical model for rendering and display.

In previously described embodiments, only a single defined path of the interventional device in the anatomical cavity is used to register the anatomical model (derived from location data) to the reference anatomical model. However, such approaches can be adapted to use a plurality of different defined paths in order to register the anatomical model.

For instance, each defined path can be associated or matched with a corresponding reference path in the reference model, e.g., using a measure of similarity or by determining the reference path that passes through a same region/area of the anatomical cavity. Registration of the two anatomical models can then be performed by defining a required rotation and/or scaling factor to be applied to the anatomical model and/or the reference anatomical model such that each defined path is (e.g., best) aligned spatially or (e.g., best) registered to its corresponding reference path.

In previously described embodiments, location data is obtained from a single interventional device. However, it is possible to obtain location measurements from each of a plurality of intervention devices, all of which can contribute to the location data. Thus, an anatomical model may be generated from location data comprising location measurements obtained using location sensors on a plurality of interventional devices.

This may be useful, for instance, in scenarios where multiple interventional devices are inserted at a same time. One example of such a scenario is during atrial fibrillation ablation procedures, in which it is common to insert an ablation catheter (for performing ablation) as well as a coronary sinus (CS) catheter, which is positioned at the coronary sinus curve, for monitoring the electrical activity of the heart during ablation.

In circumstances where there are multiple interventional devices, it is possible to define one or more sequences (and therefore paths) from any number of the interventional devices. Thus, a first sequence of location measurements may represent a path taken by a first interventional device and a second sequence of location measurement may represent a path taken by a second, different intervention device. Each sequence can be processed to identify a path taken by a respective interventional device. As before, in each sequence of location measurements, each location measurement in the sequence represents a temporally later position of the interventional device than any other location measurement earlier in the sequence.

Approaches for registering an anatomical model to a reference anatomical model using multiple paths have been previously described.

A full working example of an approach for registering an anatomical model generated from location data and a reference anatomical is hereafter provided, for the sake of improved contextual understanding. In this working example, the anatomical cavity is a cavity of the heart and surrounding cardiac system.

In atrial fibrillation ablation procedures, it is common for a catheter to be positioned in the coronary sinus to monitor the electrical activity of the heart during ablation. This catheter is commonly labelled a CS catheter. Typical examples of a CS catheter include a Deca-pole CS catheter or 20 pole CS catheter. A Deca-pole CS catheter carries 5 location sensors (here: electrodes for monitoring crossing electric fields) and the 20 pole CS catheter carries 10 location sensors (here: electrodes for monitoring crossing electric fields).

Multiple location measurements taken from a coronary sinus (CS) catheter can be used to detect the coronary sinus arc (i.e., a line that follows the coronary sinus). In particular, the tracked locations (i.e., location measurements) of the CS catheter can be used to identify or compute an arc representing a path or route taken the CS catheter through the coronary sinus. Thus, identifying the coronary sinus arc in this way identifies a path taken by the interventional device through the coronary sinus.

A reference anatomical model (e.g., a reference 3D mesh) can be automatically segmented to identify a reference path, being a path that moves through the coronary sinus represented by a part of the reference anatomical model.

This approach generates one path and identifies one reference path that can be used to register an anatomical model, produced using at least location measurements produced by one or more location sensors on the CS catheter, and the reference anatomical model.

It is possible to define one or more additional paths and reference paths that can be used in the registration of the anatomical model and the reference anatomical model.

For instance, atrial fibrillation ablation procedures also make use of an ablation catheter for performing ablation. To access the heart, for performing AF ablation procedure, it is typical for this ablation catheter to first enter through the femoral vein in the groin or from the carotid vein in the neck. Then by either going towards the head or the feet, the catheter ends up in the inferior vena cava (IVC) or the superior vena cava (SVC), respectively. The ablation catheter may carry one or more location sensors, which can be used to generate location measurements for the ablation catheter. It is possible to use these location measurements to identify a sequence of location measurements that represent a path through the vena cava.

This sequence can be used to determine a path taken by the ablation catheter through the vena cava. A reference anatomical model (e.g., a reference 3D mesh) can be automatically segmented to identify a second reference path, being a path that moves through the vena cava represented by a part of the reference anatomical model.

Registration of the anatomical model and the reference anatomical model can then be performed, using previously described approaches, using the paths and reference paths identified from the location measurements produced by the two catheters.

Fig. 6 illustrates an anatomical model registration system 600 for registering an anatomical model of an anatomical cavity, generated using location measurements provided using a location sensor carried by an interventional device and representing different positions of the interventional device within the anatomical cavity, to a reference anatomical model of the anatomical cavity.

The anatomical model registration system 600 may be the system 180 previously described, and may comprise the mapping system illustrated in Fig. 1.

In particular, the anatomical model registration system 600 may form part of a larger system 60, which also provides an embodiment.

The anatomical model registration system 600 comprises an input interface 610 configured to receive location data comprising a sequence of location measurements, wherein each location measurement in the sequence represents a temporally later position of the interventional device than any other location measurement earlier in the sequence; and receive the reference anatomical model for the anatomical cavity.

The location data may be received from the interventional device(s) 150, e.g., via the signal processor 120. Alternatively, the location data may be obtained from a memory 140.

The reference anatomical model may be received from the memory or storage unit 140. Alternatively, the reference anatomical model be received from an external source or processing unit 690, e.g., a cloud-computing system or cloud storage unit.

The anatomical model registration system 600 also comprises a data processor 620 communicatively coupled to the input interface.

The data processor 620 is configured to process the location data to generate an anatomical model of the anatomical cavity; use the sequence of location measurements to define a path taken by the interventional device; and register the generated anatomical model to the reference anatomical model using the defined path taken by the interventional device.

The anatomical model registration system may be appropriately adapted to perform any herein described method, as would be readily apparent to the skilled person.

In particular, the data processor is configured to generate output data.

As one example, the output data comprises the registered anatomical model and reference anatomical model itself.

As another example, the output data comprises registration information that defines how the anatomical model and reference anatomical model are spatially related (e.g., a rotation and/or scaling required to spatially register the two models.

As yet another example, the output data may comprise a labelled anatomical model. The data processor may produce a labelled anatomical model by labelling the anatomical model using corresponding labels for a spatially registered reference anatomical model.

The anatomical model registration system may further comprise an output interface 630, which provides the output data responsive to the registered anatomical model and reference anatomical model to one or more devices external to the anatomical model registration system 600. This information may be output, for instance, to a further data processor 690 such as a further processing module. In some examples, the information may be provided to a/the memory 140 or other storage unit.

The system 60 may comprise a user interface 190.

The user/output interface 190 may be configured to provide a visual representation of the registered anatomical model and reference anatomical model. In some examples, a display processor 195 may be configured to receive the registered anatomical model and reference anatomical model and render the anatomical models to generate display data that can be received and processed by the user/output interface 190 for providing a visual representation thereof.

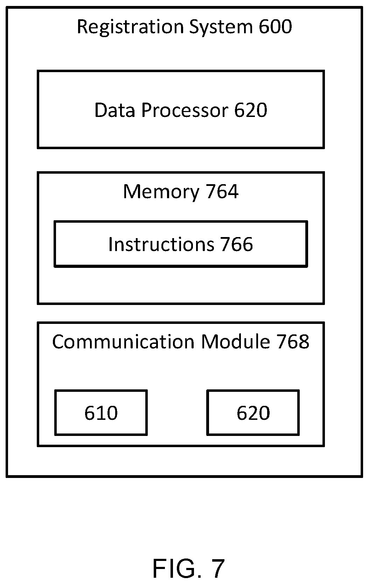

Fig. 7 is a schematic diagram of an anatomical model registration system 600, according to embodiments of the present disclosure. The anatomical model registration system 600 is configured to carry out a method according to an embodiment, such as the method 400 described with reference to Fig. 4.

As shown, the anatomical model registration system 600 may include a (data) data processor 620, a memory 764, and a communication module 768. These elements may be in direct or indirect communication with each other, for example via one or more buses.

The data processor 620 may include a central data processor (CPU), a digital signal processor (DSP), an ASIC, a controller, an FPGA, another hardware device, a firmware device, or any combination thereof configured to perform the operations described herein. The data processor 620 may also be implemented as a combination of computing devices, e.g., a combination of a DSP and a microprocessor, a plurality of microprocessors, one or more microprocessors in conjunction with a DSP core, or any other such configuration. In some embodiments, the processor is a processing system, e.g., formed of a set of distributed processors.

The memory 764 may include a cache memory (e.g., a cache memory of the data processor 620), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 764 includes a non-transitory computer-readable medium. The non-transitory computer-readable medium may store instructions. For example, the memory 764, or non-transitory computer-readable medium may have program code recorded thereon, the program code including instructions for causing the anatomical model registration system 600, or one or more components of the anatomical model registration system 600, particularly the data processor 620, to perform the operations described herein. For example, the anatomical model registration system 600 can execute operations of the method 400.

Instructions 766 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 764, with the code recorded thereon, may be referred to as a computer program product.

The communication module 768 can include any electronic circuitry and/or logic circuitry to facilitate direct or indirect communication of data between the anatomical model registration system 600, the penetration device and/or the user interface (or other further device). In that regard, the communication module 768 can be an input/output (I/O) device. In some instances, the communication module 768 facilitates direct or indirect communication between various elements of the anatomical model registration system 600.

The communications may comprise the input interface 610 and the output interface 630, for facilitating communication between the system 600 and external devices.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the Figs. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or anatomical model registration system, cause the computer or anatomical model registration system to carry out (the steps of) any herein described method. The computer program (product) may be stored on a non-transitory computer readable medium.

Similarly, there is also proposed a computer-readable (storage) medium comprising instructions which, when executed by a computer or anatomical model registration system, cause the computer or anatomical model registration system to carry out (the steps of) any herein described method. There is also proposed computer-readable data carrier having stored there on the computer program (product) previously described. There is also proposed a data carrier signal carrying the computer program (product) previously described.

The skilled person would be readily capable of developing an anatomical model registration system for carrying out any herein described method. Thus, each step of a flow chart may represent a different action performed by an anatomical model registration system, and may be performed by a respective module of the anatomical model registration system. The anatomical model registration system may be a processing system.

It will be appreciated that different disclosed processing systems may form part of a single processing system, e.g., each processing system is a sub-system of a single processing system. In the context of the present disclosure, this means that a single system could perform the actions of a mapping system and/or an anatomical model registration system and/or a field controller processing system as herein described.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". Any reference signs in the claims should not be construed as limiting the scope.

## Claims

1. An anatomical model registration system for registering an anatomical model of an anatomical cavity, generated using location measurements provided using a location sensor carried by an interventional device and representing different positions of the interventional device within the anatomical cavity, to a reference anatomical model of the anatomical cavity, the anatomical model registration system comprising:
an input interface configured to:
receive location data comprising a sequence of location measurements, wherein each location measurement in the sequence represents a temporally later position of the interventional device than any other location measurement earlier in the sequence; and
receive the reference anatomical model for the anatomical cavity;
a data processor configured to:
process the location data to generate an anatomical model of the anatomical cavity;
use the sequence of location measurements to define a path taken by the interventional device; and
register the generated anatomical model to the reference anatomical model using the defined path taken by the interventional device.

2. The anatomical model registration system of claim 1, further comprising an output interface configured to control a user interface to provide a visual representation of the generated anatomical model and the reference anatomical model, wherein the visual representation is responsive to the registration of the generated anatomical model and the reference anatomical model.

3. The anatomical model registration system of any of claims 1 to 2, wherein:
the data processor is further configured to process the reference anatomical model to identify a plurality of potential paths for an interventional device; and
the data processor is configured to register the generated anatomical model to the reference anatomical model by processing the defined path and the plurality of potential paths.

4. The anatomical model registration system of claim 3, wherein the data processor is configured to register the generated anatomical model to the reference anatomical model by:
comparing the defined path to each potential path to determine which of the plurality of potential paths best matches the defined path; and
registering the generated anatomical model to the reference anatomical model using the defined path and the best matching potential path.

5. The anatomical registration system of claim 3, wherein the data processor is configured to register the generated anatomical model to the reference anatomical model by:
defining, for each potential path, an identity of the part of the anatomical cavity through which the potential path passes;
processing the defined path to identify the part of the anatomical cavity through which the defined path passes;
identifying which of the plurality of potential paths identifies the same part of the anatomical cavity as the defined path; and
registering the generated anatomical model to the reference anatomical model using the defined path and the identified potential path.

6. The anatomical registration system of any of claims 3 to 5, wherein the anatomical cavity is a cavity in a cardiac system of a subject and the plurality of potential paths contain at least: a path through a vena cava; a path through a coronary sinus; and a path through an aortic arch.

7. The anatomical model registration system of any of claims 1 to 6, wherein the reference anatomical model is an anatomical model derived from imaging data of the anatomical cavity.

8. The anatomical model registration system of claim 7, wherein the imaging data comprises magnetic resonance imaging data; computed tomography imaging data; or 3D ultrasound imaging data.

9. The anatomical model registration system of any of claim 1 to 6, wherein the reference anatomical model is an anatomical atlas representing a population-derived anatomical model of the anatomical cavity

10. The anatomical model registration system of any of claims 1 to 9, wherein the data processor is configured to use the sequence of location measurements to define a path taken by the interventional device by:
determining a line of best fit through the sequence of location measurements, the line of best fit representing the path taken by the interventional device.

11. The anatomical registration system of claim 10, wherein the line of best fit is a line definable using a first, second or third order polynomial.

12. The anatomical registration system of any of claims 1 to 11, wherein:
the input interface is configured to receive further location measurements, each further location measurement representing a temporally later position of the interventional device than any location measurement in the sequence of location measurements; and
the data processor is configured to update the anatomical model of the anatomical cavity using the further location measurements.

13. The anatomical model registration system of any of claims 1 to 12, wherein the generated anatomical model is a polygon mesh, and optionally the reference anatomical model is a polygon mesh.

14. A computer-implemented method for registering an anatomical model of an anatomical cavity, generated using location measurements provided by a location sensor carried by an interventional device and representing different positions of the interventional device within the anatomical cavity, to a reference anatomical model of the anatomical cavity, the computer-implemented method comprising:
receiving location data comprising a sequence of location measurements, wherein each location measurement in the sequence represents a temporally later position of the interventional device than any other location measurement earlier in the sequence;
receiving the reference anatomical model for the anatomical cavity;
processing the location data to generate an anatomical model of the anatomical cavity;
using the sequence of location measurements to define a path taken by the interventional device; and
registering the generated anatomical model to the reference anatomical model using the defined path taken by the interventional device.

15. A computer program product comprising computer program code means which, when executed on a computing device having a processing system, cause the processing system to perform all of the steps of the computer-implemented method of claim 14.
